Europäisches Patentamt

(19) European Patent Office

Office européen des brevets

(11) Numéro de publication: **0 299 810**

**A1**

(12) **DEMANDE DE BREVET EUROPEEN**

(21) Numéro de dépôt: **88401181.8**

(22) Date de dépôt: **16.05.88**

(51) Int. Cl.⁴: **C 12 N 15/00**
C 12 P 21/02, A 61 K 39/00,
C 12 N 1/20
//A61K39:13,C12N9:22,
C12N9:12

(30) Priorité: **18.05.87 FR 8706925**

(43) Date de publication de la demande:
**18.01.89 Bulletin 89/03**

(84) Etats contractants désignés:
**AT BE CH DE ES FR GB GR IT LI LU NL SE**

(71) Demandeur: **INSTITUT PASTEUR**
**25-28, rue du Docteur Roux**
**F-75724 Paris Cédex 15 (FR)**

(84) Etats contractants désignés:
**AT BE CH DE ES FR GB GR IT LI LU NL SE**

(71) Demandeur: **CENTRE NATIONAL DE LA RECHERCHE SCIENTIFIQUE**
**15, quai Anatole France**
**F-75007 Paris (FR)**

(84) Etats contractants désignés: **FR**

(71) Demandeur: **INSTITUT NATIONAL DE LA SANTE ET DE LA RECHERCHE MEDICALE (INSERM)**
**101, rue de Tolbiac**
**F-75654 Paris Cédex 13 (FR)**

(84) Etats contractants désignés:
**AT BE CH DE ES FR GB GR IT LI LU NL SE**

(72) Inventeur: **Hofnung, Maurice**
**19 bis, rue Bargue**
**F-75015 Paris (FR)**

**Bedouelle, Hugues**
**40-42 rue Sébastien-Mercier**
**F-75015 Paris (FR)**

**Clement, Jean-Marie**
**25 rue Gay-Lussac**
**F-75005 Paris (FR)**

**Duplay, Pascale**
**115 rue de la Convention**
**F-75015 Paris (FR)**

**Gilson, Eric**
**25 rue Juliette-Dodu**
**F-75010 Paris (FR)**

**Leclerc, Claude**
**127 rue de Javel**
**F-75015 Paris (FR)**

**Martineau, Pierre**
**21, rue de la Bièvre**
**F-92340 Bourg-La-Reine (FR)**

**Szmelcman, Sevec**
**134 rue de Saussure**
**F-75017 Paris (FR)**

**Van der Werf Sylvie**
**1, rue Jacques Coeur**
**75004 Paris (FR)**

(74) Mandataire: **Peaucelle, Chantal et al**
**S.C. Ernest Gutmann - Yves Plasseraud 67, boulevard Haussmann**
**F-75008 Paris (FR)**

Le titre de l'invention a été modifié (Directives relatives à l'examen pratiqué à l'OEB, A-III, 7.3)

(54) **Procédé de préparation de polypeptides par ADN recombinant.**

㊗ L'invention concerne un procédé de fabrication, éventuellement d'exportation, et de purification d'un polypeptide déterminé. Il comprend l'étape consistant à provoquer l'expression dans un organisme-hôte, notamment une bactérie E. coli, d'un acide nucléique recombinant codant pour un polypeptide hybride contenant une séquence codant pour la protéine MalE ou MalX, et une séquence codant pour le polypeptide recherché, la récupération du polypeptide hybride par chroma-tographie affine sur une colonne d'amylose et la récupération du polypeptide par élution à l'aide d'une solution de maltose.

L'invention concerne également les applications des polypeptides hybrides ainsi obtenus, notamment dans le domaine de la production de compositions de vaccin en utilisant les protéines MalE ou MalX en tant que polypeptides vecteurs pour la présentation d'épitopes.

EP 0 299 810 A1

**Description**

## PROCEDE DE PRODUCTION D'UN POLYPEPTIDE DETERMINE DANS UN ORGANISME-HOTE TRANSFORME PAR UN ADN RECOMBINANT CONTENANT DES SEQUENCES NUCLEIQUES CODANT POUR CE POLYPEPTIDE ET POUR UNE PROTEINE AFFINE POUR UN OSE. APPLICATIONS, NOTAMMENT POUR LA PRODUCTION DE COMPOSITIONS DE VACCIN.

L'invention est relative à un procédé de production d'un polypeptide ou d'une protéine ayant des propriétés choisies déterminées, par culture d'un organisme-hôte préalablement transformé par un ADN recombinant contenant des séquences codant pour ce polypeptide et pour une protéine affine pour un ose. Une forme préférée du procédé selon l'invention comporte une étape supplémentaire de purification de ce polypeptide comprenant la mise en contact de ce polypeptide avec un ose insolubilisé.

L'invention concerne également des vecteurs contenant l'ADN recombinant sus-mentionné, ainsi que des cultures de cellules transformées par ces vecteurs, et leurs utilisations, notamment pour la production de compositions de vaccin.

Plusieurs techniques ont été décrites pour faire produire un polypeptide ou une protéine par des organismes-hôtes préalablement transformés par un vecteur approprié lui-même modifié par une séquence d'insertion contenant un acide nucléique codant pour cette enzyme ou cette protéine, convenablement placée sous le contrôle d'éléments de régulation, notamment d'un promoteur, autorisant l'expression de cet acide nucléique dans l'organisme hôte transformé. Une fois le gène (ou le vecteur le contenant) mis au point, il devient une source permanente de polypeptides.

Cette approche génétique pour la conception de nouveaux polypeptides possède plusieurs inconvénients : le polypeptide peut être dégradé in vivo, être toxique pour la cellule-hôte ou difficile à purifier à partir d'extraits bactériens. Il a déjà été proposé de contourner le problème de la dégradation, notamment lorsque l'hôte cellulaire choisi est E. coli en fusionnant le polypeptide étranger à une protéine stable, par exemple à la β-galactosidase, qui sert de vecteur. La fusion d'un polypeptide une protéine vecteur, via leurs gènes codants, peut également faciliter la purification de ce polypeptide si la protéine vecteur conserve son activité dans l'hybride, et si elle est facile à purifier, par exemple au moyen d'une chromatographie d'affinité. La β-galactosidase, la protéine A de S. aureus et des immunoglobulines ont été utilisées à cette fin. L'introduction, à la jonction entre les deux séquences nucléiques, de séquences spécifiques, permet le clivage du polypeptide greffé après purification de la protéine hybride.

D'autres solutions ont également été proposées. On citera pour mémoire les techniques dans lesquelles la séquence codant pour le polypeptide étranger a été fusionnée à une séquence signal visant à permettre l'exportation du polypeptide étranger hors du cytoplasme de l'hôte cellulaire, pour éviter l'effet toxique possible du polypeptide étranger à l'égard de l'hôte cellulaire. Il a également été proposé d'obvier à l'effet toxique possible du polypeptide étranger, en plaçant le gène codant sous le contrôle d'un promoteur normalement réprimé, ce promoteur étant ensuite déréprimé dans la phase terminale de croissance de la culture cellulaire pour alors induire la synthèse du polypeptide étranger.

Une autre difficulté souvent rencontrée réside dans la conservation de l'activité du polypeptide étranger ainsi produit, par exemple lorsque ce polypeptide consiste en une enzyme. A cet égard, la technique susmentionnée consistant à fusionner la séquence codant pour le polypeptide étranger avec une séquence signal a également, dans certains cas, été proposée pour obtenir le repliement normal du polypeptide étranger dans un milieu moins réducteur que le cytoplasme cellulaire, notamment lorsque l'hôte cellulaire utilisé appartient à une espèce bactérienne. Mais jusqu'à ce jour, il n'a pas souvent été possible de remédier à ces difficultés, surtout lorsque le polypeptide étranger s'avérait être d'une taille très importante. Enfin les tentatives de promouvoir le transport d'une protéine cytoplasmique, à partir du cytoplasme vers la région périplasmique des bactéries transformées dans les conditions sus-indiquées, a en général échoué, la protéine cytoplasmique restant coincée ou bloquée dans les membranes mêmes des bactéries.

De même, les purifications finales ont jusqu'à ce jour été difficiles à réaliser. Par exemple, dans le cas de protéines hybrides, il n'est pas toujours facile de séparer les protéines hybrides correctement repliées - et actives - des protéines hybrides non correctement repliées et inactives qui ont pu être produites simultanément par le même organisme-hôte.

En outre, dans le cas des purifications de protéines produites par des techniques de chromatographie ou analogue, mettant en jeu des molécules affines pour les protéines à purifier, fixées sur un support solide adéquat, il n'est pas toujours exclu que certaines de ces molécules affines ne soient pas séparées en même temps que les protéines sélectivement retenues, au cours des opérations d'élution finale. Même si les proportions de molécules affines ainsi séparées peuvent n'être qu'extrêmement faibles, elles peuvent contribuer à "souiller" le produit final de façon néfaste, eu égard aux applications dont les protéines ainsi purifiées peuvent ensuite être l'objet. On conçoit aussi que la préparation des colonnes d'affinités elles-mêmes, pour ces purifications, sera souvent délicate et coûteuse, surtout lorsque la réduction à un minimum des inconvénients précédemment mentionnés s'avérera constituer un impératif essentiel.

L'invention a pour but de fournir un procédé de génie génétique qui permette de remédier, mieux qu'il n'est souvent possible à ce jour, aux difficultés plus particulièrement rappelées ci-dessus, tout en étant d'une application aisée, relativement peu coûteuse et très efficace, pour produire un polypeptide contenant une chaîne peptidique possédant elle-même des propriétés choisies, susceptibles d'être ensuite complètement exprimées.

Ainsi l'invention concerne un procédé de fabrication et de purification d'un polypeptide contenant une chaîne peptidique déterminée possédant elle-même des propriétés choisies, qui comprend une étape consistant à provoquer l'expression dans un organisme-hôte approprié transformé et mis en culture à cet effet, d'un acide nucléique recombinant à phase de lecture ouverte comprenant une séquence codant pour la chaîne peptidique et une ou plusieurs séquences distinctes codant pour une ou plusieurs parties du polypeptide vecteur, et une étape de récupération du polypeptide hybride exprimé par l'acide nucléique recombinant sous la forme d'un extrait à partir du susdit hôte cellulaire et une étape de purification, caractérisé en ce que le polypeptide vecteur est une protéine affine pour au moins un ose déterminé, en ce que lesdites "une ou plusieurs séquences codant pour une ou plusieurs parties du polypeptide vecteur " sont telles que les propriétés de transport hors du cytoplasme et d'affinité pour l'ose déterminé du polypeptide vecteur intact sont conservées dans le susdit polypeptide hybride, en ce que l'étape de purification comprend la mise en contact de l' extrait du polypeptide hybride avec un ose correspondant sous une forme insolubilisée compatible avec l'affinité mutuelle de la protéine affine et de l'ose, notamment un ose polymérisé, et en ce que l'on récupère, après séparation des produits non fixés sur l'ose, le polypeptide hybride contenant ladite chaîne peptidique déterminée.

Avantageusement, le procédé de l'invention comprend également une étape supplémentaire de récupération de ladite chaîne peptidique déterminée à partir du polypeptide hybride et qui sera développée plus loin.

Selon un mode de réalisation du procédé de l'invention, l'acide nucléique recombinant comprend une séquence nucléique codant pour le polypeptide vecteur placée toute entière en aval, ou en amont, de la séquence nucléique codant pour la chaîne peptidique déterminée.

Avantageusement, l'acide nucléique recombinant comprend une première séquence nucléique codant pour une première partie du polypeptide vecteur placée en amont de la séquence nucléique codant pour la chaîne peptidique déterminée, et une seconde séquence nucléique codant pour une seconde partie du polypeptide vecteur, placée en aval de cette dernière séquence, les première et seconde parties sus-mentionnées représentant la totalité du polypeptide vecteur lorsqu'elles sont réunies. Les conditions dans lesquelles la séquence nucléique codant pour la chaîne peptidique déterminée est introduite entre deux parties de la séquence nucléique codant pour le polypeptide vecteur sans pour autant modifier les fonctions de transport hors du cytoplasme du polypeptide vecteur ainsi modifié, ni l'affinité de ce dernier pour l'ose déterminé, sont plus particulièrement décrites dans la demande de brevet européen n° 87.400 504.4 déposée le 6 mars 1987 ou les demandes de brevet correspondantes déposées dans d'autres pays et dans (11). Les sites situés sur la séquence nucléique codant pour le polypeptide vecteur, et au niveau desquels la séquence nucléique codant pour la chaîne peptidique déterminée peut être insérée sans pour autant modifier les fonctions caractéristiques du polypeptide vecteur précisées ci-dessus, sont désignés dans ce qui suit par l'expression "sites permissifs".

Un polypeptide vecteur particulièrement préféré est constitué par la protéine MalE.

La protéine MalE est une protéine de 370 acides aminés qui est codée par le gène malE d'E. coli. Son promoteur, malEp, est un promoteur fort qui est activé par la protéine MalT en présence de l'inducteur maltose et réprimé par le glucose. Ces contrôles opèrent encore lorsque malE et son promoteur sont portés par un plasmide à copies multiples, comme ppD1, dont la fabrication a été décrite dans (1).

MalE est une protéine abondante. Elle est exportée dans le périplasme bactérien par l'intermédiaire d'un peptide signal N-terminal qui est clivé durant l'exportation (2). MalE est une protéine affine pour le maltose et les maltodextrines, qui est nécessaire au transport de ces sucres à travers l'enveloppe bactérienne. MalE, de même que son précurseur, peut être purifié en une étape par chromatographie d'affinité sur amylose pontée par traitement à l'épichlorhydrine. La protéine est éluée par compétition avec du maltose 10 mM (3).

Il a également été montré que cette protéine pouvait être modifiée en certains de ses sites, sans pour autant perdre certaines de ses activités. On se reportera notamment à l'article de DUPLAY et coll. (4). Cet article et tous les autres documents identifiés à la fin de ce texte doivent être considérés comme faisant partie intégrante de cette description, pour autant que leurs contenus respectifs servent à la compléter, quant à la nature et à la structure des matières premières à partir desquelles l'invention a été produite.

A cet égard, il est rappelé que DUPLAY et coll. ont inséré au hasard un adaptateur ("linker") BamHI dans le gène malE d'un plasmide pPD1 qui le contenait. Deux des plasmides modifiés par ces insertions, malE91 et malE140, changent le cadre de lecture et conduisent à la synthèse de protéines modifiées possédant 362 (respectivement 369) acides aminés N-terminaux de MalE et une extension C-terminale de 12 (respectivement 27) acides aminés. Une troisième insertion, malE127, dans la séquence signal, empêche l'exportation de la protéine MalE dans le périplasme (4).

L'invention fournit donc un procédé particulièrement efficace pour produire par génie génétique et purifier toute chaîne peptidique ou protéine déterminée, et ceci grâce à un vecteur recombinant obtenu par construction, dans lequel une séquence nucléique codant pour ladite protéine a été placée en aval de la séquence codant pour le polypeptide vecteur, notamment pour la protéine MalE, elle-même placée sous le contrôle d'un promoteur efficace.

La construction du vecteur recombinant utilisé dans le procédé sus-mentionné de l'invention, peut également être réalisée de manière à ce que la séquence nucléique codant pour la protéine déterminée soit située en amont de la séquence d'ADN codant pour le polypeptide vecteur, notamment pour la protéine MalE,

3

et en aval d'un promoteur efficace de l'expression de la séquence d'ADN codant pour ladite protéine suivie de celle codant pour MalE.

Selon un mode de réalisation du procédé de l'invention, l'organisme-hôte utilisé pour cette fabrication consiste en une bactérie E. coli, cas dans lequel il est avantageux d'avoir recours au gène malE lui-même, lequel comprend alors également le promoteur endogène correspondant malEp. Dans l'hypothèse qui précède, l'étape de purification comprendra la mise en contact avec l'ose insolubilisé correspondant à la protéine MalE, avantageusement une maltodextrine insolubilisée ou une amylose pontée obtenues dans les conditions déjà rappelées plus haut. L'utilisation du couple de caractéristiques : 1. production de la protéine recherchée à l'aide d'un vecteur contenant la séquence codante fusionnée au gène malE - 2. purification par mise en contact avec du maltose ou du glucose amené à l'état polymérisé, est avantageuse à bien des égards. Au niveau de la production, on met à profit la capacité du gène malE à s'exprimer de façon abondante dans E. coli et simultanément la capacité du glucose ou du maltose insolubilisé à permettre la séparation des seules protéines hybrides ayant conservé la capacité affine de la protéine MalE naturelle ou "sauvage". Il est remarquable que l'on peut grâce à ces vecteurs également obtenir une protéine hybride, dans laquelle la partie correspondant à la protéine recherchée conserve l'activité de cette dernière, cette activité se manifestant directement ou après restitution, comme cela sera montré dans les exemples décrits plus loin, avec des protéines déterminées de grande taille, telles que des nucléases (150 aminoacides) et le fragment Klenow d'une polymérase (650 aminoacides) utilisés à titre de modèles expérimentaux.

Il importe naturellement que la séquence nucléique codant pour la protéine recherchée soit insérée dans une région convenable du gène malE, pour que soient conservées au sein de la structure de cette dernière les propriétés affines pour le maltose de la protéine MalE et l'activité recherchée pour la protéine déterminée devant être synthétisée. A cette fin, il est avantageux que la séquence nucléique codant pour la protéine déterminée soit placée en aval, ou en amont, de la séquence codant pour la protéine MalE vis-à-vis du sens de la transcription du gène recombinant. Cette condition n'est cependant nullement imposée. La détermination de la localisation la plus avantageuse de l'insertion de la séquence codant pour la protéine recherchée dans le gène malE peut être déterminée, notamment par la technique d'insertion au hasard de la séquence codant pour une chaîne peptidique déterminée, décrite dans la demande de brevet européen sus-mentionnée.

A la capacité de purification tout à fait remarquable que permet le système selon l'invention, s'ajoute encore la très grande souplesse des vecteurs préférés envisagés ci-dessus, en ce que l'on peut choisir la région de la bactérie dans laquelle la protéine hybride produite est transportée, soit dans le cytoplasme, soit dans le périplasme (du moins lorsque la nature même de la protéine hybride n'interdit pas le transport dans cette dernière région). Ce choix est sous le contrôle de l'utilisateur, grâce à la possibilité qu'il a de s'assurer à volonté du transport de la protéine hybride formée, soit dans le périplasme (pour autant que ce transport soit possible pour les raisons évoquées ci-dessus) en mettant à profit les propriétés spécifiques à cet égard de la séquence signal normalement intercalée dans le gène malE entre la séquence codante et le promoteur, soit en s'assurant que de toute façon la protéine hybride ne sera pas transportée au-delà de la région cytoplasmique, grâce à l'introduction d'une mutation dans cette séquence signal, mutation dont l'effet sera précisément d'empêcher l'exportation de la protéine hybride dans le périplasme. Dans le premier cas, la protéine hybride pourra être aisément récupérée à partir du périplasme, notamment en soumettant les bactéries transformées à un choc osmotique, par exemple dans les conditions décrites dans (4). Dans l'autre cas, la protéine hybride sera récupérée à partir du cytoplasme après rupture complète des parois bactériennes, par exemple à l'issue d'un traitement avec des détergents ou à l'aide de moyens mécaniques ou enzymatiques bien connus de l'homme du métier. Cette deuxième alternative certes d'emploi moins pratique sera néanmoins préférée, dès lors que l'on souhaitera protéger la bactérie contre le "stress" qui pourrait être engendré du fait de la tendance naturelle au transport de la protéine hybride dans le périplasme sous l'effet de la séquence signal non mutée, quand bien même ce transport s'avèrerait difficile en raison même de la structure hybride.

De façon remarquable, le procédé selon l'invention permet - pour celles des protéines qui sont effectivement transportables à travers la membrane bactérienne séparant les régions cytoplasmique et périplasmique de l'organisme-hôte - l'exportation dans le périplasme de protéines normalement cytoplasmiques, donc avec un grand coefficient de purification, avant toute mise en contact avec l'ose insolubilisé, ou mise en oeuvre de toute autre technique de purification supplémentaire.

A cet égard l'invention concerne donc plus particulièrement, à titre de variante, le procédé de production et de purification sus-défini, dans lequel l'étape de purification par contact avec l'ose-insolubilisé est omise, et ce plus particulièrement lorsque la chaîne peptidique recherchée consiste en tout ou partie d'une protéine cytoplasmique, autrement dit en une protéine non excrétée par son hôte naturel ; et ce plus particulièrement dans le cas où l'organisme-hôte est une bactérie.

Un autre avantage encore du système préféré décrit ci-dessus réside dans le caractère inductible ou réprimable à volonté du promoteur malEp, dont les capacités d'activation en présence de l'inducteur maltose et de répression en présence de glucose ont déjà été rappelées plus haut. Il est en particulier avantageux de réaliser la croissance des cultures bactériennes préalablement transformées en présence de doses de glucose n'interférant pas avec la capacité de développement des cultures, néanmoins suffisantes à éviter l'expression du gène recombinant, la culture étant ensuite soustraite à l'action du glucose, lorsqu'elle a atteint le stade de développement voulu et que l'expression du gène recombinant, avec pour conséquence la production de la protéine hybride, devient souhaitée. Dans ces conditions, on protège la culture bactérienne en cours de développement contre l'éventuel effet toxique de la protéine hybride formée, celle-ci n'étant produite en quantités importantes qu'au terme du stade de développement d'abord recherché de la culture.

Il est d'ailleurs avantageux de réaliser toutes ces opérations à une température inférieure à 37°C, notamment à une température comprise entre 28°C et 35°C, par exemple voisine de 30°C. L'effet toxique éventuel de la protéine hybride se trouve alors amoindri.

Bien qu'il soit avantageux d'avoir recours au gène malE naturel pour la construction de l'ADN recombinant, il apparaîtra immédiatement à l'homme du métier que ces conditions ne sont nullement limitatives. En particulier, le promoteur malEp peut être remplacé par construction par tout autre promoteur efficace reconnu par la bactérie. En particulier, on peut avoir recours à tout promoteur fort dont l'activité constitutive peut être réprimée ou encore à tout promoteur fort inductible par le réglage approprié des conditions de culture des bactéries transformées. A titre d'exemple de promoteurs qui pourraient ainsi être substitués par construction au promoteur malEp, pour obtenir une expression dans E. coli, on mentionnera les promoteurs PL et PR du phage λ, le promoteur tac inductible par l'IPTG (isopropyl-β-thiogalactoside), les promoteurs de l'opéron lactose, de l'opéron tryptophane, des promoteurs mixtes dérivés des précédents, etc..

Il est entendu que l'expression "séquence codant pour la protéine MalE" doit être considérée comme concernant aussi bien la séquence codant pour la protéine sauvage que pour des protéines MalE modifiées, sous réserve que l'affinité caractéristique pour l'ose correspondant soit conservée. Il est tout aussi clair que cette expression concerne également les séquences d'acides nucléiques qui différeraient des précédentes, par des substitutions de nucléotides n'altérant pas la structure de la protéine correspondante. De ce point de vue l'invention concerne également l'utilisation des séquences d'acides nucléiques produites partiellement ou totalement de façon synthétique, en mettant en jeu la dégénérescence du code génétique.

Outre les bactéries Gram⁻, telle que E. coli sus-mentionnnée, tout autre hôte utilisable dans les techniques du génie génétique peut ainsi être utilisé, dès lors que les vecteurs utilisables pour la transformation sont disponibles ou peuvent être conçus. On mentionnera à titre d'exemple d'autres types de bactéries dans laquelle MalE peut être utilisé comme support pour purifier des protéines, les bactéries Gram⁺, par exemple B. subtilis, ou les corynebactéries ou encore des levures ou des cellules eucaryotes supérieures, notamment de mammifères. Naturellement la séquence codant pour la protéine MalE devra dans chaque cas être placée par construction sous le contrôle d'un promoteur approprié à son expression dans l'organisme-hôte. Dans chaque cas, l'opération de purification qui suit permet de séparer efficacement les protéines hybrides recherchées, grâce à leur affinité pour l'ose insolubilisé correspondant.

Concernant l'expression de MalE chez les bactéries Gram⁺, les inventeurs ont en effet mis en évidence que la protéine MalE, présente une forte homologie avec la protéine MalX de Streptococcus pneumoniae . Cette protéine MalX est inductible par le maltose, et joue probablement un rôle dans la capture des maltodextrines, telles que le maltotetraose, chez les bactéries Gram⁺ (12,13). MalX est une protéine liée à la membrane des bactéries Gram⁺, à la différence de MalE qui se situe dans la périplasme des bactéries Gram⁻. La protéine MalX est synthétisée sous forme d'un précurseur, exportée hors du cytoplasme, et la transformation de la cystéine amino-terminale en un acide aminé lipophile est probablement responsable de l'ancrage de MalX à l'extérieur de la membrane cytoplasmique des bactéries Gram⁺. Une étude comparative entre les protéine MalE et MalX sera plus particulièrement détaillée dans les exemples qui suivent.

L'expression de la protéine MalE dans les cellules eucaryotes sera plus particulièrement détaillée dans les exemples décrits plus loin. L'utilisation de cellules eucaryotes offre notamment l'avantage de fabriquer des protéines nécessitant, par leur fonction, d'être glycosylées, ou de posséder des ponts disulfure, ou des protéines difficilement exportées chez les bactéries.

Dans ce qui précède, l'invention a été décrite plus particulièrement en rapport avec la séquence nucléique codant pour une protéine MalE. L'invention n'est cependant pas limitée à l'utilisation de vecteurs recombinants incluant une séquence nucléique codant pour cette protéine. L'invention s'étend également à l'utilisation de toute autre protéine-vecteur, dès lors qu'elle présente une affinité marquée pour un ose. On mentionnera à titre d'exemple l'utilisation, pour la production des ADNs recombinants nécessaires à la production de la protéine déterminée recherchée, la séquence nucléique codant pour la protéine MalX affine pour le maltose chez les bactéries Gram⁺, des séquences nucléiques codant pour les protéines affines de l'arabinose, du galactose, du ribose, etc., les opérations de purification correspondantes comprenant alors la mise en contact ultérieure des protéines hybrides ainsi produites avec les oses respectivement amenés dans un état insolubilisé, notamment par polymérisation, le cas échéant à l'aide d'un agent de réticulation, tel que l'épichlorhydrine ou tout autre agent permettant à la fois la réalisation de cette polymérisation et la préservation des affinités mutuelles de l'ose et de la protéine affine pour cet ose.

A cet égard, il faut encore souligner l'avantage essentiel que représente l'utilisation de colonnes d'ose insolubilisé, alors fonctionnelles comme colonnes de chromatographie affine, au niveau de l'innocuité postérieure des produits qui peuvent en être séparés par des techniques d'élution usuelles, après avoir été au préalable fixés de façon sélective sur ces colonnes. Les produits constitutifs de ces colonnes sont normalement dépourvus de toute toxicité et leur utilisation sera d'un intérêt tout particulier pour la production de protéines ou de polypeptides ayant des applications en médecine humaine ou vétérinaire.

En outre la capacité de rétention sélective de ces colonnes est très importante puisque c'est la matrice même des produits insolubilisés mis en oeuvre qui possède l'affinité pour le polypeptide vecteur sélectivement lié au produit recherché, contrairement aux colonnes de chromatographie d'affinité classiques, dans lesquelles la matière constitutive de la colonne est formée d'un matériau support, normalement inerte à l'égard des protéines ou polypeptides à isoler, sur lequel ont au préalable été fixées des molécules ou produits présentant l'affinité requise vis-à-vis du produit à séparer.

Les polypeptides ou protéines hybrides peuvent ensuite être récupérées à partir de l'ose insolubilisé, notamment par élution à l'aide d'une solution combinant un principe permettant leur déplacement. Cette solution contient de préférence l'ose soluble correspondant, en l'occurrence du maltose, lorsque le polypeptide vecteur est dérivé de la protéine MalE, ou MalX.

Préalablement à l'étape de purification sur colonne d'ose des protéines hybrides, ces dernières sont récupérées à partir du périplasme des bactéries Gram⁻ (comme il l'a été précisé ci-dessus dans le cas de l'utilisation de MalE), ou au niveau des membranes des cellules hôtes après lavage par une solution appropriée, voire encore à partir de cytoplasme desdites cellules hôtes après rupture des membranes de ces dernières. Il faut préciser que pour les protéines hybrides contenant un polypeptide vecteur, tel que la protéine MalX, restant ancrées à la surface de la membrane des bactéries Gram⁺, il est particulièrement avantageux de prévoir l'insertion d'une sous-séquence nucléique codant pour une séquence d'aminoacides spécifiquement clivable par une enzyme déterminée, ou par un procédé chimique, au niveau aminoterminal de la protéine MalX (notamment en aval de la cystéine aminoterminale sus-mentionnée). Le traitement de la membrane cellulaire à l'aide de l'enzyme ou du procédé chimique clivant spécifiquement la séquence d'aminoacides sus-mentionnée, conduit à la libération de la protéine hybride. La protéine hybride contenant MalX chez les bactéries Gram⁺, peut également être directement obtenue par modification de la partie aminoterminale de la séquence nucléique codant pour MalX, de manière à ce que la protéine hybride ne se fixe pas à la surface de la membrane de la bactérie.

Dans la description qui précède il n'a été évoqué que le cas de la purification des protéines hybrides contenant le polypeptide ou la protéine recherchés.

Il faut d'ailleurs souligner que la protéine hybride peut former elle-même le polypeptide recherché, chaque fois que l'on souhaitera bénéficier de la conservation de propriétés propres du polypeptide vecteur ou encore, dans le cas de polypeptides ou de protéines immunogènes, de l'effet adjuvant d'immunité que peut apportée la proteine affine pour un ose, notamment MalE ou MalX. Cet aspect est d'un intérêt tout particulier lorsque le polypeptide recherché présente un poids moléculaire relativement faible, incompatible avec une immunogénicité suffisante in vivo dudit polypeptide recherché. A cet égard il pourra là encore être fait usage de la technique décrite dans la demande de brevet européen n° 87.4005044 ou dans (11) pour déterminer le site approprié d'introduction (ou site permissif) de la séquence codant pour le polypeptide recherché dans la séquence codante du gène malE oumalX, de façon à simultanément conserver dans la protéine hybride les propriétés affines de la protéine MalE ou MalX pour l'ose insolubilisé et assurer l'exposition convenable du polypeptide recherché au sein de la protéine hybride, pour que celle-ci possède les propriétés immunogènes recherchées du polypeptide.

A cet égard, l'invention concerne des compositions immunogènes dirigées contre des antigènes (notamment des antigènes pathogènes susceptibles d'être neutralisés par des anticorps), caractérisées par l'association, avec un véhicule approprié à la production de compositions de vaccin, d'un polypeptide hybride obtenu par transformation, selon le procédé de l'invention de bactéries Gram⁻, ou Gram⁺, ou de cellules eucaryotes, à l'aide d'un acide nucléique recombinant constitué d'une séquence nucléique codant pour un polypeptide affine pour un ose, notamment pour MalE ou MalX, cette dernière séquence nucléique contenant au niveau d'un de ses sites permissifs, la séquence d'ADN codant pour un épitope déterminé.

L'invention concerne plus particulièrement une composition immunogène dirigée contre le SIDA, caractérisée en ce que le polypeptide hybride qui la compose contient un peptide anti-HIV, notamment un fragment actif du récepteur T4, inséré dans MalE, ledit polypeptide hybride étant associé avec un véhicule approprié à la production de compositions de vaccin.

Des protéines hybrides entre MalE et des polypeptides possédant des caractères immunologiques, tels que le récepteur T4 situé sur les lymphocytes T4, et constituant la cible pour les virus de type HIV caractéristiques du SIDA, ainsi que l'épitope C₃ de la protéine VP1 du poliovirus, seront plus particulièrement décrites dans les exemples de réalisation de l'invention qui suivent.

L'invention n'est nullement limitée à la purification de protéines ou polypeptides hybrides. En particulier, la purification peut être conduite dans des conditions permettant la séparation de la chaîne peptidique déterminée (ou encore de la protéine recherchée) du polypeptide vecteur placé en amont, ou en aval, dans la protéine hybride, vis-à-vis du sens de traduction, voire même la récupération de la seule protéine recherchée, dès lors qu'elle se trouverait placée entièrement en aval, ou en amont, du polypeptide vecteur, dans la mesure où par construction une courte séquence nucléique (ou sous-séquence) codant pour une séquence d'aminoacides spécifiquement clivable par une enzyme déterminée ou un procédé chimique avait auparavant été intercalée dans l'acide nucléique recombinant, entre la séquence codant pour le polypeptide vecteur et la séquence codant pour la protéine recherchée. Le cas échéant, d'autres sous-séquences semblables à celle mentionnée ci-dessus, peuvent être insérées dans l'acide nucléique recombinant, notamment de manière à ce que la séquence codant pour la chaîne peptidique déterminée soit intercalée entre deux de ces sous-séquences, pour mieux isoler encore la chaîne peptidique déterminée.

L'opération d'élution de la protéine recherchée comprend alors le traitement de l'ose insolubilisé, sur lequel a au préalable été fixée la protéine hybride, par le traitement clivant spécifiquement la séquence d'aminoacides, le polypeptide vecteur restant alors fixé sur l'ose.

En variante, après récupération du polypeptide hybride à partir de l'ose insolubilisé, on traite le polypeptide hybride en solution par l'enzyme spécifique ou le traitement chimique spécifique, on remet en contact les produits de clivage de la protéine hybride avec l'ose insolubilisé et on récupère le polypeptide alors non fixé consistant essentiellement en la chaîne d'acides aminés recherchée.

Il va de soi que le polypeptide recherché peut également être séparé de la protéine hybride, même s'il se trouve incorporé entre deux fragments du polypeptide vecteur au sein de l'hybride, comme il l'a été décrit ci-dessus. Dans ce cas de figure, l'acide nucléique recombinant mis en oeuvre dans le procédé selon l'invention, est construit de manière à ce qu'il contienne deux sous-séquences codant pour une séquence d'aminoacides sélectivement clivable par une enzyme spécifique et placées respectivement en amont et en aval de la séquence nucléique codant pour la chaîne peptidique déterminée.

A titre d'exemple de séquence nucléique susceptible d'être insérée, notamment entre les première et seconde parties de la séquence codante, on mentionnera par exemple la séquence :

ATCGAGGGTAGG

IleGluGlyArg

laquelle est clivable sélectivement par le facteur Xa, dont l'intervention dans la coagulation du sang est bien connue. Il va de soi que l'utilisation de cette séquence particulière ne peut être envisagée qu'à condition que la protéine recherchée ne contienne pas de séquence tétrapeptidique supplémentaire. On mentionnera encore à titre d'autre exemple de séquences d'acides aminés ainsi sélectivement clivables, les séquences qui apparaissent dans le tableau I qui suit et dans lequel ont été citées des séquences caractéristiques ainsi que les enzymes permettant leur clivage au niveau de l'acide aminé repéré à l'aide d'une petite flèche verticale.

### TABLEAU I

côté de l'acide aminé
C-terminal

| | |
|---|---|
| Entérokinase | Asp Asp Asp Lys↓ |
| Collagénase | Pro X↓Gly Pro Y↓(Pro Val↓Gly Pro) |
| Trypsine | Lys↓ et Arg↓ |
| Clostridiopeptidase B | Arg↓(Lys)↓ |
| Protéase staphy-lococcique | Asp↓ et Glu↓ |
| Thermolysine | ↓Leu, ↓Ile, ↓Phe et↓Val (et autres) |
| Chymotrypsine | Phe↓,Trp[L] et Tyr↓ |
| Pepsine | Phe↓, Trp↓ et Tyr↓ (et autres) |

On peut également avoir recours à des clivages chimiques spécifiques au niveau des acides aminés ou des dipeptides qui suivent.

### TABLEAU II

côté de l'acide aminé
C-terminal

| | |
|---|---|
| Bromure de cyanogène | Met↓ |
| Acide formique | Asp↓Pro |
| Amine hydroxyle | Asn↓Gly |
| Réactifs oxydants | Trp↓ |
| 2-Nitro-5-thiocya-nobenzoate | ↓Cys |

Le choix de la séquence mise en oeuvre dépendra de la structure même du polypeptide dont l'intégrité devra être conservée à l'issue de cette opération de purification.

**0 299 810**

L'invention concerne également des cultures de cellules portant, notamment au niveau de leur surface externe, un épitope caractéristique reconnaissable par le système immunitaire de l'organisme dans lequel les susdites cellules sont susceptibles d'être introduites, caractériséeS en ce que lesdites cellules sont transformées à l'aide d'un acide nucléique recombinant à phase de lecture ouverte comprenant une séquence nucléique codant pour ledit épitope insérée au niveau d'un site permissif d'une séquence nucléique codant pour une protéine affine pour un ose.

Avantageusement, la protéine affine pour un ose sus-mentionnée est la protéine MalE ou MalX.

Parmi les cellules sus-mentionnées portant au niveau de leur surface externe un épitope, on distinguera notamment les bactéries Gram⁻ (par exemple E. coli), les bactéries Gram⁺ (par exemple B. subtilis), et les cellules eucaryotes.

L'insertion de la séquence codant pour ledit épitope au niveau d'un site permissif de la séquence codant pour la protéine MalE, tout en conservant les propriétés de transfert hors du cytoplasme et d'affinité pour le maltose de ces dernières, permet à la protéine hybride contenant l'épitope, et qui est codée par l'acide nucléique sus-mentionné, d'être localisée au niveau du périplasme des bactéries Gram⁻, lorsque ces dernières sont utilisées en tant que cellule-hôtes, faisant de ces cellules des réactifs de choix pour la présentation d'un épitope caractéristique dans l'organisme dans lequel lesdites cellules sont susceptibles d'être introduites.

D'une manière générale, l'utilisation des protéines MalE et MalX en tant que polypeptides vecteurs pour la pésentation d'épitopes, peut être envisagée chez les bactéries Gram⁻, Gram⁺ ou les cellules eucaryotes.

L'invention a également pour objet des compositions immunogènes dirigées contre des antigènes (notamment des antigènes pathogènes neutralisables par des anticorps dirigés contre un épitope déterminé), et caractérisées par l'association des cellules sus-mentionnées (vivantes ou tuées, notamment par la chaleur, dans le cas des bactéries) portant au niveau de leur surface externe ledit épitope, avec un véhicule pharmaceutiquement approprié à la production de compositions de vaccin.

L'invention concerne également un acide nucléique recombinant à phase de lecture ouverte comprenant une séquence nucléique codant pour la chaîne peptidique déterminée et une ou plusieurs séquences distinctes codant pour une ou plusieurs parties du polypeptide vecteur, caractérisé en ce que lesdites "une ou plusieurs séquences codant pour une ou plusieurs parties du polypeptide vecteur " sont telles que les propriétés de transport dans le cytoplasme et d'affinité pour l'ose déterminé du polypeptide vecteur intact, sont conservées dans le polypeptide hybride exprimé par ledit acides nucléique recombinant, et en ce que lesdites séquences nucléiques sont sous le contrôle d'éléments de régulation, notamment de la promotion et de la terminaison de la transcription, reconnus par les bactéries Gram⁺ ou par les cellules eucaryotes.

Avantageusement, lesdites "une ou plusieurs séquences distinctes codant pour une plusieurs parties du polypeptide vecteur" sont les séquences nucléiques codant pour tout ou partie des protéines MalE ou MalX.

L'invention concerne également un acide nucléique recombinant tel que défini ci-dessus caractérisé en ce que le polypeptide vecteur est la protéine MalX, et en ce que lesdits éléments de régulation sont reconnus par les bactéries Gram⁻.

Selon divers modes de réalisation de l'invention, les acides nucléiques recombinants sus-mentionnés sont construits de telle sorte que :
- la séquence nucléique codant pour MalE et MalX est placée toute entière en amont de la séquence d'ADN codant pour la chaîne peptidique déterminée,
- la séquence nucléique codant pour MalE et MalX est placée toute entière en aval de la séquence d'ADN codant pour la chaîne peptidique déterminée,
- la séquence nucléique codant pour la chaîne peptidique déterminée est insérée au niveau d'un site permissif de la séquence nucléique codant pour MalE et MalX.

Des sous-séquences codant pour une séquence d'aminoacides clivable par une enzyme spécifique, ou par un traitement chimique, peuvent également être insérées entre les parties des acides nucléiques recombinants sus-mentionnés codant respectivement pour la partie MalE ou MalX et pour la chaîne peptidique déterminée, comme il l'a été précédemment décrit.

L'invention concerne également les acides nucléiques recombinants sus-mentionnés en tant que vecteurs, notamment de type plasmidique, aptes à se répliquer chez les bactéries Gram⁻ ou Gram⁺ ou chez les cellules eucaryotes.

Des caractéristiques préférées de l'invention aparaîtront encore au cours de la description qui suit de constructions préférées de l'invention, schématiquement représentées dans les fig. 1,2,3 et 5, sans que celles-ci puissent être considérées comme ayant un caractère limitatif.

Il sera également fait référence dans ce qui suit à la figure 4 représentant l'expression de constructions MalE-T4 et T4-MalE chez E. coli, les figures 6 à 8 représentant l'expression de MalE chez des cellules eucaryotes, et la figure 9 représentant les homologies entre MalE et MalX.

Les plasmides qui ont été mis en oeuvre dans ces constructions ont déjà fait l'objet de descriptions antérieures. On se reportera notamment :
- à la publication de VIERA et MESSING, 1982, pour ce qui est du plasmide pUC9,
- à la publication de WILLIAMS et NEUBERGER, 1986, pour la description du fragment BamHI-polAK-BamHI qui code pour le fragment de Klenow de l'ADN polymérase I,
- à l'article de NEUBERGER et al, 1984, pour la description du phage M13Mp8 recombinant qui contient le gène nuc de la nucléase de S. aureus,

- à la publication de P. DUPLAY et al, J. Mol. Biol., 194, 663-673, 1987, pour ce qui est des plasmides pPD91, pPD127 et pPD140, dont il sera question ci-après.

1) Fabrication d'un plasmide codant pour la protéine hybride MalE91-Klenow, exprimant à la fois l'activité de MalE et l'activité de l'enzyme Klenow.

L'hybride entre malE etpolAK a été construit de la façon suivante.

Le fragment BamHI-polAK décrit dans NEUBERGER 1986, repris à partir du plasmide pUC9 recombinant obtenu, a été inséré dans le plasmide pPD91, plus particulièrement dans le linker BamHI de celui-ci, lui-même contenu dans le gène malE, au niveau du triplet codant pour le 362ème acide aminé de MalE. Cette insertion devait donc avoir pour résultat de placer le gène polAK en aval de la plus grande partie du gène malE. L'orientation de polAK dans les plasmides recombinants obtenus a été déterminée par double digestion BglII-SacI. Celui des plasmides dans lequel polAK se trouvait correctement orienté a été dénommé pHB11. Celui-ci code pour la protéine MalE91-Klenow.

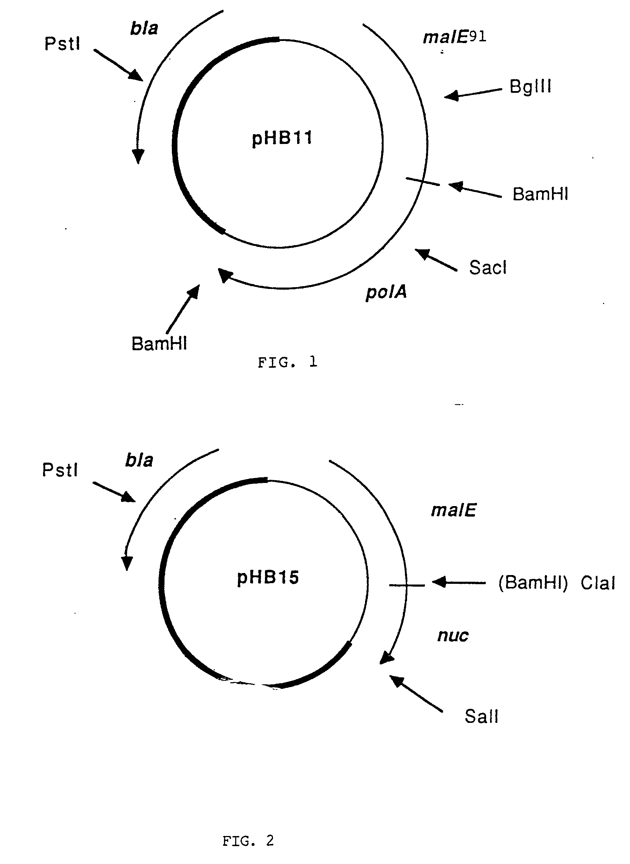

Le plasmide pHB11 est schématiquement représenté dans la fig. 1. Le site BamHI, représenté dans la partie droite de la figure, sépare la partie du gène codant pour la partie distale de MalE91 et la partie proximale du gène polA codant pour l'enzyme Klenow.

A partir de pHBII, il a été obtenu un plasmide pHB12 portant une mutation dans la séquence signal du gène malE. A cet effet, on a effectué un échange par recombinaison génétique entre les fragments PstI-BglII de pHB11, qui comportait cette séquence néanmoins dépourvue de mutation avec la séquence PstI-BglII correspondante issue du plasmide pPD127 décrit dans (4), qui contenait une telle mutation. pHB12 code pour la protéine hybride malE127/91-Klenow.

2) Fabrication d'un plasmide codant pour une protéine hybride MalE140-Nuc, exprimant à la fois une activité MalE et une activité de nucléase.

Un fragment (BamHI-nuc-SalI) codant pour la nucléase de S. aureus a été excisé du phage MP8 recombinant décrit dans NEUBERGER et al, 1984, et substitué, par ligation dans pBR322, en lieu et place du fragment BamHI-SalI initialement contenu dans celui-ci. Le plasmide obtenu a été dénommé pHB13.

Par échange du fragment EcoRI-BamHI de pHB13, immédiatement en amont du fragment (BamHI-nuc-SalI), avec le fragment (EcoRI-(malEp-malE)-BamHI) du plasmide pDP140, on a obtenu le plasmide pHB14, dans lequel le gènenuc était alors placé en aval du gène malE. Ces deux gènes ont été mis en phase :
- par remplissage des extrémités cohésives du site unique BamHI contenu dans pHB14, en présence des quatre déoxynucléotides et de polymérase de Klenow.

pHB15 code alors pour une protéine hybride MalE140-nucléase.

Le plasmide pHB15 est schématiquement représenté sur la figure 2. Le site (BamHI)ClaI dans la partie droite de la figure, sépare la partie du gène codant pour la partie distale de MalE et la partie proximale du gènenuc codant pour la nucléase sus-mentionnée.

3) Production et purification des protéines hybrides MalE91-Klenow et MalE140-Nucléase par des cultures de E. coli transformées au préalable par les plasmides pHB11, pHB12 et pHB15, respectivement.

La souche PD28, un dérivé ∆malE444,malTᶜ1, recA::tn10 de MC4100 de E. coli (4) a été utilisée comme hôte pour l'expression des gènes hybrides. La délétion ∆malE444 du gène malE, qui est non polaire sur l'expression des gènes distaux de l'opéron mal E-malF-malG, évite l'expression de la protéine MalE sauvage à partir du chromosome bactérien et sa copurification avec les hybrides sur MalE. ∆malE444 rend la souche PD28 défective pour le transport du maltose. La mutation malTᶜ 1 permet l'activation constitutive du promoteur malEp en l'absence de maltose inducteur. Pour éviter le risque de léthalité de l'expression constitutive de protéines hybrides pour la cellule, on a fait croître tous les dérivés de PD28 en milieu complet additioné de 0,4 à 2 % de glucose. Dans ces conditions le promoteur malEp est fortement réprimé, sans que soit entravé le développement de la culture sur un milieu complet. Un lavage des cellules et leur mise en culture dans un milieu complet dépourvu de glucose, éventuellement additionné de maltose, permet alors d'obtenir l'expression de MalE ou de protéines hybrides à partir de ces plasmides dès la première génération. On effectue toutes les croissances à 30°. Des expériences de contrôle ont montré que l'expression ou l'exportation en grande quantité de MalE à partir d'un plasmide sont abolies à 42°.

Quand ils sont introduits dans la souche PD28, les plasmides pPD1, pPD91, pPD140 et PHB15 provoquent l'expression, en quantité importante, des protéines MalE, MalE91, MalE140 et de l'hybride MalE140-Nucléase. On trouve ces quatre protéines dans la fraction cellulaire libérée partir du périplasme, lorsque les bactéries productrices sont soumises à un traitement visant à provoquer un choc osmotique.

Les plasmides pPD127, pHB11 et pHB12 provoquent l'expression, en quantités importantes, de la protéine MalE127 et des hybrides MalE91-Klenow et MalE127/91-Klenow dans la fraction cellulaire soluble.

Il est à remarquer qu'une certaine proportion de MalE91-Klenow a également été exportée dans le périplasme (produite par le plasmide pHB11 qui ne portait aucune mutation dans la séquence signal du gènemalE).

Les protéines MalE sauvage, MalE91, MalE140 et MalE127 (protéines témoins) et les protéines hybrides MalE140-Nucléase, MalE91-Klenow et MalE127/91-Klenow, respectivement obtenues à partir des cultures bactériennes correspondantes, ont été purifiées par chromatographie d'affinité sur amylose pontée dans les conditions décrites dans (4). Dans les cas de MalE sauvage, MalE140-Nucléase et MalE91-Klenow, cette purification a été faite à partir d'extraits périplasmiques. Dans les cas de MalE127 et MalE127-Klenow, la purification a été faite à partir d'un extrait soluble de cellules entières.

Dans chaque cas, on a traité une culture de 500 ml ensemencée à une densité optique, mesurée à 600 nm, égale à 0,1 et récoltée à une densité égale à 1. Chacun des extraits (environ 140 mg de protéine pour les extraits solubles, obtenue à partir des bactéries entières, et environ 14 mg pour les extraits périplasmiques), étant repris dans 25 ml d'un tampon (par exemple 50 mM Tris-Cl pH 7,5 ; 2,5 mM β-mercapto-éthanol ; 0,1 PMSF), ont été déposés sur une colonne de 6 ml de résine ; les colonnes ont été lavées avec environ 5 volumes de tampon, puis les protéines ont été éluées au moyen de maltose 10 mM.

Les quantités retenues et éluées ont été égales à 1,76 mg pour MalE sauvage, 1,64 mg pour l'hybride MalE140-Nucléase, et 1,16 mg pour MalE127. Les colonnes n'étaient pas saturées puisque MalE et l'hybride MalE140-Nucléase n'ont pas été retrouvés dans les fractions qui avaient passé au travers des colonnes sans être retenues.

En revanche seule une faible proportion (68 μg) de l'hybride MalE127/91-Klenow devait être retenue sur la colonne dans les mêmes conditions, ce qui suggère que, dans le cas particulier de cette protéine hybride, la portion polymérase de Klenow perturbe le repliement correct de la portionmalE de celle-ci.

On a obtenu 172 μg de protéine hybride MalE91-Klenow purifiée à partir de l'extrait périplasmique, après chromatographie d'affinité dans les conditions sus-indiquées.

On a trouvé que l'activité de l'hybride MalE140-Nucléase était égale à 2100 unités nucléase/mg d'hybride, c'est-à-dire 8942 u/mg de la portion nucléase de l'hybride. Par comparaison, l'activité d'une préparation commerciale de nucléase (Boehringer) était égale à 2580 u/mg. Par conséquent, la nucléase a sa pleine activité dans l'hybride. La souche PD28 (pHB15) a un phénotype Mal$^+$ sur milieu MacConkey maltose à 30°. Par conséquent, le gène hybride malE140-nuc est capable de compléter la délétion ΔmalE444 pour la fermentation du maltose et l'hybride MalE140-Nucléase est au moins partiellement actif pour le transport du maltose. En résumé, l'hybride MalE140-Nucléase est exporté dans le périplasme et conserve les activités de ses deux partenaires.

On a trouvé que l'activité de l'hybride MalE127/91-Klenow était égale à 4514 unités polymérase/mg d'hybride, c'est-à-dire 7431 u/mg de la portion fragment de Klenow de l'hybride. Par comparaison, l'activité d'une préparation commerciale de fragment de Klenow (Boehringer) était à égale 9445 u/mg. Par conséquent, le fragment de Klenow a sa pleine activité dans l'hybride. En résumé, l'hybride MalE127/91-Klenow est une protéine cytoplasmique qui possède l'activité polymérase et au moins certaines des propriétés de MalE.

On a trouvé que l'activité de l'hybride MalE91-Klenow était égale à 3100 unités polymérase par mg d'hybride, c'est-à-dire 5100 unités/mg de la portion fragment de Klenow de l'hybride. Par conséquent, le fragment de Klenow conserve également son activité dans l'hybride MalE91-Klenow.

En résumé l'hybride MalE91-Klenow peut être reportée dans le périplasme, tout en conservant l'activité polymérase et certaines des propriétés de malE.

Les préparations de protéines étaient pures à 95 %, comme l'ont montré des analyses par électrophorèse en gel de polyacrylamide-SDS. L'homme de métier appréciera que ces protéines auraient pu être purifiées davantage encore, par des moyens classiques, par exemple par dia-lyse.

### 4) Fabrication de plasmides codant pour les protéines hybrides MalE-T4 et T4-MalE.

Les sites accepteurs pour le gène T4 sont d'une part le site BamHI inséré en C-terminal de malE dans le plasmide pPD140 (utilisé pour la fusion MalE-T4) et d'autre part le site BamHI inséré en N-terminal de MalE dans le plasmide pPD127 (utilisé pour la fusion T4-MalE).

Des polynucléotides synthétiques ont été dans une première étape insérés dans les sites accepteurs sus-mentionnés de façon à introduire de nouveaux sites compatibles avec les fragments T4 à insérer, accorder les phases de lecture des deux gènes, au besoin restituer les parties manquantes ou introduire des codons stop ou fin de construction. Les plasmides pMEC et pMEN ont été ainsi respectivement obtenus à partir des plasmides pPD140 et pPD127.

Pour la réalisation de la fusion MalE-T4, le fragment Fnu4HI (contenant le gène T4 mais pas la séquence signal (ss) ni les parties transmembranaire et cytoplasmique C-terminales) décrit dans MADDON et al, Cell (1985) 42 93-104, a été inséré dans le plasmide pMEC, plus particulièrement au niveau du site Xba introduit lors de la première étape dans celui-ci. Lé gène T4 est placé en aval du gène MalE dans le plasmide pMET ainsi obtenu.

Pour la réalisation de la fusion T$_4$-MalE, le fragment HpaII (contenant le gène T4 et la quasi-totalité de la séquence signal, mais pas les parties transmembranaire (mb) et cytoplasmique (cyt) C-terminales) décrit dans la référence mentionnées ci-dessus, a été inséré dans le plasmide pMEN, plus particulièrement au niveau du site EcoRI introduit lors de l'étape précédente dans celui-ci. Le gène T4 est placé en amont du gène MalE dans le plasmide pTME ainsi obtenu.

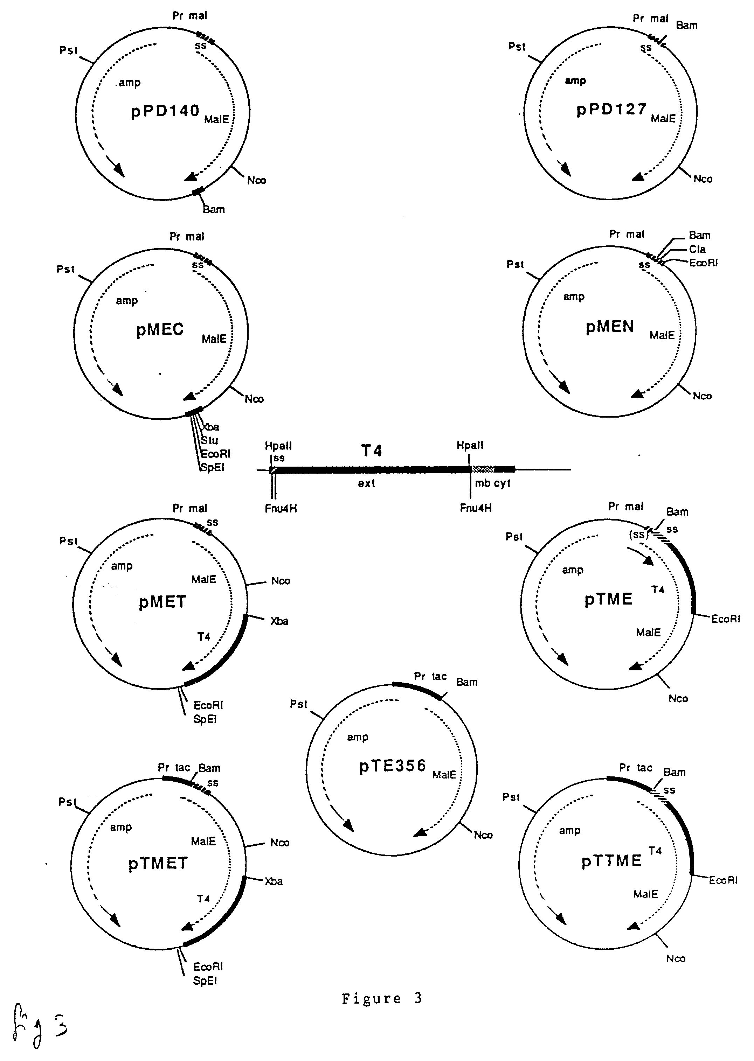

Les plasmides pD140, pPD127, pMEC, pMEN, pMET, pTME, sus-mentionnés contiennent le promoteur naturel de malE (Pr mal, inductible par le maltose, sensible à la repression catabolique), et sont indiqués sur la figure 3.

Les plasmides pTE356 (construit par insertion d'un fragment EcoRI-BamHI contenant le promoteur tac du plasmide pDR340, commercialisé par la société PHARMACIA, dans le plasmide pPD356 décrit dans DUPLAY et al, (1987) J.Mol.Biol.194, 663-673), pTMET et pTTME obtenus à partir de ce dernier selon le principe précédent, contiennent respectivement les gènes malE, malE-T4 et T4-malE sous contrôle du promoteur tac (inductible par l'IPTG, insensible à la repression catabolique), et sont également représentés sur la figure 3.

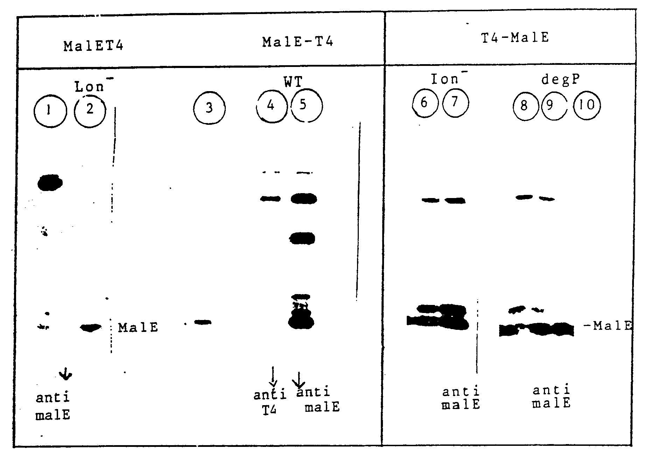

Des expériences d'immunoprécipitation (figure 4) permettent d'observer que les deux constructions MalE-T4 et T4-MalE sont exprimées chez E.coli et sont plus ou moins dégradées selon la souche utilisée.

Les colonnes numérotées de 1 à 10 correspondent aux souches d'E.coli contenant les constructions MalE-T4, T4-MalE répartis de la façon suivante :

souches :
lon⁻ :    1,2,6,7
wt :    4,5
degP :    8,9

Constructions :
MalE-T4 :    1,4,5
T4-malE :    6,7,8,9

Témoins :
MalE :    2,3,10

Les colonnes 1 à 3, et 6 à 10, correspondent à l'immunoprécipitation d'extraits bruts révélés par des anticorps anti-MalE.

Les colonnes 4 et 5 correspondent à l'immunoprécipitation d'extraits radioactifs révélés par des anticorps anti T4 (OKT4A) (colonne 4), et par des anticorps anti MalE (colonne 5).

## 5) Utilisation de la protéine MalE comme vecteur de présentation d'épitope

L'épitope $C_3$ de la protéine VP1 du poliovirus a été inséré génétiquement dans deux sites de la protéine MalE selon la méthode décrite dans la demande de brevet européen n° 87.400.504.4. Les protéines recombinantes retiennent les principales caractéristiques de la protéine sauvage: exportation dans le périplasme, rétention sur une colonne d'amylose, complémentation d'une délétion chromosomique du gène. Une étude de l'immunogénicité de ces protéines a été entreprise chez la souris Balb/c.

Les souris ont été immunisées avec des bactéries vivantes, des bactéries tuées par la chaleur, des lysats bactériens obtenus par sonication et des préparations semi-purifiées (choc osmotique. Protéines de 50 à 70 % pures). Deux voies d'immunisation ont été testées : intraveineuse et sous cutanée.

Par voie i.v., toutes les préparations ont donné de très forts titres anti-peptide de $10^4$ à plus de $10^6$ par voie s.c., seule la préparation de bactéries tuées par la chaleur a donnée une réponse anti-peptide (supérieure à $10^5$). Les sérums ont été testés pour leur capacité à immunoprécipiter des particules virales natives et dénaturées par la chaleur : tous les sérums donnant des titres supérieurs à $10^4$ précipitent les particules dénaturées à 100 %. Les sérums ont été testés pour la neutralisation in vitro et donnent des titres du 1/8 au 1/64 corrélés avec les réponses anti-peptides et les résultats d'immunoprécipitations.

## 6) Expression de la protéine MalE dans les cellules eucaryotes

Le fragment BamHI-ACCI comprenant le gène malE du plasmide pTE356 précédemment cité a été inséré dans le vecteur pSUTK Moβ (décrit dans NICOLAS et BERG (1983), Cold Spring Harbor, Symposium Quantitative Biology, 469-485) par traitement de ce dernier à l'aide de BglII + NruI.

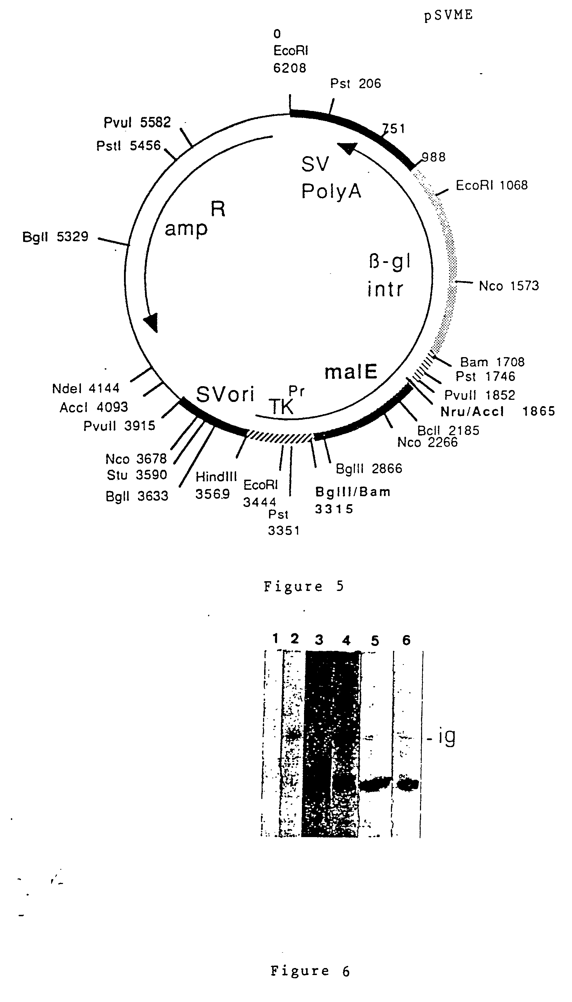

Le plasmide pSVME ainsi obtenu est représenté sur la figure 5. Ce plasmide contient le gène malE ainsi que les éléments génétiques rendant possible son expression dans les cellules eucaryotes (promoteurs du virus SV40 et du gène de la thymidine kinase du virus de l'herpès, régions introniques du gène T de SV40 et de la β-globine de lapin, site de polyadénylation de l'ARN précoce de SV40). Ces constructions ont été introduites dans des cellules en culture (fibroblastes embryonnaires de hamster chinois transformés par SV40 ou cellules L de souris) par transfection (technique du précipité de phosphate de calcium). Une synthèse importante de matériel immunoprécipitable par des anticorps dirigés contre MalE a été mise en évidence par technique d'immunotransfert (Western Blot) (figure 6). MalE ainsi synthétisée coexiste sous deux formes. L'une est de la même taille que la protéine MalE synthétisée dans les bactéries (forme mature). L'autre est d'une taille légèrement supérieure compatible avec celle du précurseur de MalE (avant le découpage de sa séquence signal). La protéine MalE est exportée dans le milieu de culture où on la retrouve essentiellement sous sa forme mature. La fraction extraite à partir des cellules est en revanche composée en majorité par l'espèce la plus lourde.

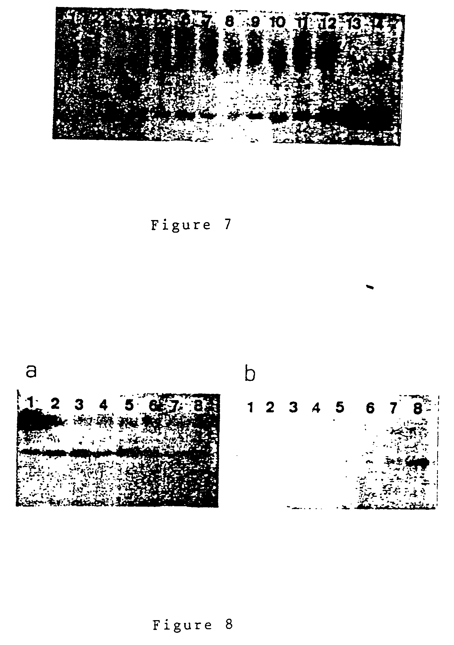

Des lignées cellulaires exprimant MalE de façon stable ont été établies (figure 7). Ce résultat a été obtenu en cotransfectant les cellules par les constructions MalE et une construction conférant aux cellules la résistance à la généticine (G418). La quasi-totalité des clones résistants expriment MalE en quantités variables selon les clones tout en gardant des propriétés de croissance normales. MalE est, comme dans les expériences d'expression transitoire, exportée dans le milieu de culture.

Il a également été mis en évidence que la protéine MalE exprimée dans des cellules eucaryotes est purifiable en une étape sur colonne d'amylose (figure 8).

Puisque la protéine MalE est exprimée et exportée dans des cellules eucaryotes, l'introduction dans les cellules eucaryotes des constructions permettant l'expression de protéines hybrides, en particulier des fusions entre le gène malE et le gène récepteur des lymphocytes T4, pour les purifier en une étape, conduit à un type de technologie présentant plusieurs avantages :

a) repliement correct des protéines eucaryotes qui ne sont pas nécessairement exportées avec succès par les bactéries. En effet, les modifications post traductionnelles, indispensables pour l'activité biologique de certaines protéines eucaryotes (glycosylations, formation exacte de ponts disulfures etc..) ne sont pas réalisées chez les bactéries (Ferenci, T. et al, (1978) F.E.B.S. Letters 94, 213-217) ;

b) il est possible d'obtenir des clones stables produisant les protéines hybrides ;

c) il est possible d'augmenter l'expression des protéines hybride en ajoutant, lors de l'introduction de l'ADN recombinant dans les cellules, des constructions permettant l'amplification génique des structures intégrées (par exemple le gène dhfr dont l'amplification peut être sélectionnée par la résistance au methotrexate).

d) Les protéines seront purifiées en une étape à partir des surnageants de cultures cellulaires par fixation sur colonne d'amylose et élution par le maltose.

e) On peut créer artificiellement des sites de clivage au niveau de la jonction entre MalE et la protéine désirée, par exemple des sites spécifiques pour la collagénase (GERMINO J. et al, (1984), proc. Natl. Acad. Sci., USA, 81, 4692-4696). Le produit de l'hydrolyse pourrait être débarassé de la fraction contenant MalE (ainsi que des produits non clivés) par la rétention de ces derniers sur colonne d'amylose.

Les techniques et les résultats des expérimentations illustrées par les figures 6, 7 et 8 sus-mentionnées sont les suivants :

-Figure 6

Technique : les cellules CO60 de hamster ont été transfectées avec le plasmide pSVME.

Après 5 jours, les protéines synthétisées par ces cellules ont été analysées (Western Blot) (lignes 3 à 5). Des cellules non transfectées ont été utilisées comme témoin (lignes 1 et 2). Les cellules (lignes 1, 3 et 4) ainsi que les surnageants de culture (lignes 2 et 5) ont été analysées. Dans les lignes 2,4,5 et 6 les extraits ont été immunoprécipités par un anticorps anti-MalE avant leur dépôt sur gel SDS-PAGE (dans ces conditions on voit les immunoglobulines notées ig.) Dans les lignes 1 et 3 les extraits cellulaires ont été déposés directement. Le résultat du transfert est révélé par un anticorps anti-MalE. Ligne 6 : protéine MalE purifiée à partir d'extraits bactériens.

Résultats : Les cellules CO60 n'expriment pas de protéines reconnues par un anticorps dirigé contre malE (cellules : ligne 1 ou surnageant : ligne 2). Les cellules transfectées par pSVME synthétisent MalE (lignes 3 et 4) sous 3 formes : l'une est de la taille de MalE (ligne 6) l'autre est plus lourde. MalE est exportée dans le milieu de culture (ligne 5) sous la forme mature (ligne 6).

Par conséquent MalE est vraisemblablement synthétisée sous forme de précurseur dans les cellules (forme lourde) et exportée dans le milieu de culture.

Figure 7

Technique : les cellules CO60 ont été transfectées avec le plasmide pSVME mélangé au plasmide RSVneo. Des clones résistants au G418 ont été sélectionnés et les surnageants de culture analysés (immunoprécipitation, SDS-PAGE, transfert sur nitrocellulose, révélation par un anticorps anti-MalE, protéine A iodée 125I). Lignes 1 à 12 : clones résistants au G418. Lignes 13 et 14 : MalE purifiée à partir d'extraits bactériens.

Résultats : la quasi totalité des clones exportent MalE dans le milieu de culture, le niveau d'expression étant variable (à la limite de la détection : clones 2,4 et 5 ou en abondance : clones 3 et 12).

Il est donc possible d'obtenir des clones stables exprimant et exportant MalE.

Figure 8

Technique : des surnageants de cultures cellulaires ( en a : CO60 et en b : clone 12-cf figure 7) ont été déposés sur colonne d'amylose. La colonne a été lavée et éluée par le maltose 100 mM. En a (expérience de reconstitution), 1 μg de MalE purifiée à partir d'extraits bactériens a été mélangé au surnageant de cellules CO60 avant de charger la colonne. 8 fraction ont été recueillies dans chaque cas après élution.

Résultats :a : l'expérience de reconstitution montre que l'on retrouve la protéine MalE éluée par le maltose dans les conditions expérimentales utilisées. La colonne est probablement surchargée car MalE se retrouve dès les premières fractions. Il est probable qu'un lavage plus extensif aurait abouti à un profil d'élution plus conforme aux résultats habituels.b : la protéine MalE synthétisée par le clone 12 est retenue sur colonne d'amylose et éluée par le maltose (fraction 8).

Ainsi, il est possible de purifier la protéine MalE synthétisée par les cellules de Hamster sur colonne d'amylose. Les protéines fusionnées à MalE synthétisées par les cellules eucaryotes sont également purifiables par cette technique.

En conclusion, MalE possède plusieurs avantages comme protéine vecteur sur les autres systèmes existants. C'est une protéine bactérienne ; elle est de taille moyenne et monomérique, à volonté périplasmique ou cytoplasmique : elle est exprimée par les cellules eucaryotes, et est facilement purifiable par chromatographie d'affinité sur un support d'affinité très peu coûteux; on peut l'éluer de la résine dans des conditions physiologiques, compatibles avec la conservation de pleines activités des polypeptides ou protéines synthétisés. De plus elle permet d'exporter dans l'espace périplasmique des protéines normalement cytoplasmiques.

La capacité de ces dernières à être transportées dans la région périplasmique pourra être chaque fois appréciée expérimentalement. Il peut encore être souligné que la capacité de transport dans cette région peut être interprétée comme tenant à la très grande efficacité à cet effet de la protéine MalE et à la possibilité que ce transport est peut-être effectué avant même que le repliement correct de la protéine ait été achevé.

## 7) Homologie entre la protéine MalX de Streptococcus pneumoniae et la protéine MalE de E. coli

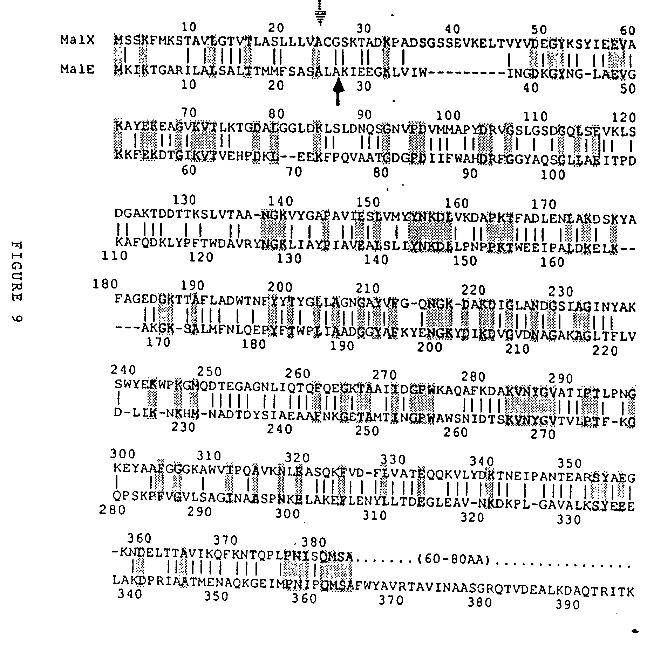

Les résultats de cette étude sont indiquées sur la figure 9 dans laquelle :
- les résidus identiques de MalE et MalX sont indiqués par des régions grises ;
- les résidus homologues entre deux protéines sont indiqués par des barres verticales ;
- la flèche noire indique le site de clivage du précurseur de MalE ;
- la flèche grise indique le site de clivage probable de MalX ;
- environ 60 à 80 acides aminés constituent la partie C-terminale de MalX.

L'homologie entre MalE et MalX est très prononcée (31 % de résidus identiques sur 334 acides aminés, et l'homologie atteint environ 75 % si l'on ne fait pas de distinction entre résidus identiques et homologues)

Les plasmides pHB11 et pHB15 ont été déposés à la Collection Nationale des Cultures de Micro-Organismes (CNCM) de l'INSTITUT PASTEUR de Paris, le 18 mai 1987, sous les n° I-662 et I-663 respectivement. Les plasmides pSVME, pTME, pTMET, pTTME ont été déposés à la COLLECTION NATIONALE DES CULTURES DE MICRO-ORGANISMES le 10 mai 1988 sous les numéros I-759, I-760, I-761, I-762 respectivement, et le pMET a été déposé le 16 mai 1988 sous le numéro I-763.

## BIBLIOGRAPHIE

1. DUPLAY, P., BEDOUELLE, H., FOWLER, A., ZABIN, I., SAURIN, W., HOFNUNG, M. 1984, J. Biol. Chem., 259, 10606-10613.
2. BEDOUELLE, H., BASSFORD, P.J., Jr., FOWLER, A.V., ZABIN, I., BECKWITH, J. et HOFFNUNG, M. 1980, Nature, 285, 78-81.
3. FERENCI, T. et KLOTZ, U 1978, F.E.B.S. Letters, 94, 213-217.
4. DUPLAY, P., SZMELCMAN, S., BEDOUELLE, H. et HOFNUNG, M. 1987, J. Mol. Biol., 194, 663-673.
7. JOYCE, C.R. et GRINDLEY, N D.F. 1984. J. Bacteriol., 158, 636-643.
8. VIEIRA, J. et MESSING, J. 1982, Gene, 19, 259-268.
9. NEUBERGER, M.S., WILLIAMS, G.T. et FOX, R.O. 1984.
10. WILLIAMS G.I. et NEUBERGER M.S. 1986, Gene 43, 319-324.
11. CHARBIT, A. et al, 1986, Embo J., vol. 5, n° 11, 3029-3037.
12. LACKS, S.A, SUNN, J.J., & GREENBERG, B. Cell,31, 7-12 (1981)
13. LACKS, S.A., Genetics,60, 685-706, (1968).

## Revendications

1. Procédé de fabrication et de purification d'un polypeptide contenant une chaîne peptidique déterminée possédant elle-même des propriétés choisies, qui comprend une étape consistant à provoquer l'expression dans un organisme-hôte approprié transformé et mis en culture à cet effet, d'un acide nucléique recombinant à phase de lecture ouverte comprenant une séquence codant pour la chaîne peptidique et une ou plusieurs séquences distinctes codant pour une ou plusieurs parties du polypeptide vecteur, et une étape de récupération du polypeptide hybride exprimé par l'acide nucléique recombinant sous la forme d'un extrait à partir du susdit hôte cellulaire et une étape de purification, caractérisé en ce que le polypeptide vecteur est une protéine affine pour au moins un ose déterminé, en ce que lesdites "une ou plusieurs séquences codant pour une ou plusieurs parties du polypeptide vecteur " sont telles que les propriétés de transport hors du cytoplasme et d'affinité pour l'ose déterminé du polypeptide vecteur intact, sont conservées dans le susdit polypeptide hybride, en ce que l'étape de purification comprend la mise en contact de l' extrait du polypeptide hybride avec un ose correspondant sous une forme insolubilisée compatible avec l'affinité mutuelle de la protéine affine et de l'ose, notamment un ose

## 0 299 810

polymérisé, et en ce que l'on récupère, après séparation des produits non fixés sur l'ose, le polypeptide hybride contenant ladite chaîne peptidique déterminée.

2. Procédé selon la revendication 1, caractérisé en ce qu'il comprend une étape supplémentaire de récupération de la chaîne peptidique déterminée, ayant les susdites propriétés choisies, par séparation à partir du polypeptide hybride.

3. Procédé selon la revendication 1 ou 2, caractérisé en ce que l'acide nucléique recombinant comprend une séquence nucléique codant pour le polypeptide vecteur placée toute entière en aval de la séquence nucléique codant pour la chaîne peptidique déterminée.

4. Procédé selon la revendication 1 ou 2, caractérisé en ce que l'acide nucléique recombinant comprend une séquence nucléique codant pour le polypeptide vecteur placée toute entière en amont de la séquence nucléique codant pour la chaîne peptidique déterminée.

5. Procédé selon la revendication 1 ou 2, caractérisé en ce que l'acide nucléique recombinant comprend une séquence nucléique codant pour la chaîne peptidique déterminée, et insérée au niveau d'un site permissif de la séquence nucléique codant pour le polypeptide vecteur.

6. Procédé selon les revendications 2 et 3, caractérisé en ce que l'acide nucléique recombinant contient une sous-séquence codant pour une séquence d'aminoacides sélectivement clivable par une enzyme spécifique ou un traitement chimique spécifique et placée entre les parties de l'acide nucléique recombinant codant respectivement pour le susdit polypeptide vecteur et ladite chaîne peptidique, et, le cas échéant, une séquence semblable placée en aval de la séquence codant pour le polypeptide vecteur.

7. Procédé selon les revendications 2 et 4, caractérisé en ce que l'acide nucléique recombinant contient une sous-séquence codant pout une séquence d'aminoacides sélectivement clivable par une enzyme spécifique et placée entre les parties de l'acide nucléique recombinant codant respectivement pour le susdit polypeptide vecteur et ladite chaîne peptidique, et, le cas échéant, une séquence semblable placée en aval de la séquence codant pour ladite chaîne peptidique.

8. Procédé selon les revendications 2 et 5, caracterise en ce que l'acide nucléique recombinant contient deux sous-séquences codant pour une séquence d'aminoacides sélectivement clivable par une enzyme spécifique ou un traitement chimique spécifique et placées respectivement en amont ou en aval de la séquence nucléique codant pour ladite chaîne peptidique déterminée.

9. Procédé selon l'une quelconque des revendications 2 à 8, caractérisé en ce que l'on récupère la chaîne peptidique déterminée ayant les susdites propriétés choisies à partir des produits fixés sur l'ose, par traitement de ces derniers avec l'enzyme spécifique ou le traitement chimique spécifique.

10. Procédé selon l'une quelconque des revendications 2 à 8, caractérisé et en ce que, après récupération du polypeptide hybride à partir de l'ose insolubilisé, on traite le polypeptide hybride en solution par l'enzyme spécifique ou le traitement chimique spécifique, en ce que l'on remet en contact les produits de clivage de la protéine hybride avec l'ose insolubilisé et en ce que l'on récupère le polypeptide alors non fixé consistant essentiellement en la chaîne peptidique recherchée.

11. Procédé selon l'une quelconque des revendications 1 à 10, caractérisé en ce que l'acide nucléique recombinant est sous le contrôle d'un promoteur réprimable par un composé susceptible d'être apporté de l'extérieur à l'organisme-hôte, notamment dans son milieu de culture, et en ce que l'on réalise dans un premier temps la culture de l'organisme-hôte préalablement transformé avec la susdite séquence nucléique en présence de ce composé, avant de soustraire la culture à l'action de ce composé dans des conditions permettant l'expression de la susdite séquence nucléique.

12. Procédé selon l'une quelconque des revendications 1 à 11, caractérisé en ce que le polypeptide vecteur est constitué par une protéine affine pour le maltose et en ce que l'ose insolubilisé est un polymère insoluble de $\alpha(1\text{-}4)$ glucose ou de l'amylose.

13. Procédé selon la revendication 12, caractérisé en ce que le polypeptide vecteur est une protéine affine pour le maltose chez les bactéries Gram⁻.

14. Procédé selon la revendication 13, caractérisé en ce que le polypeptide vecteur est·la protéine affine de maltose de E. coli (MalE).

15. Procédé selon la revendication 12, caractérisé en ce que le polypeptide vecteur est une protéine affine pour le maltose chez les bactéries Gram⁺.

16. Procédé selon la revendication 15 , caractérisé en ce que le polypeptide vecteur est la protéine affine pour le maltose chez Streptococcus pneumoniae (MalX).

17. Procédé selon l'une quelconque des revendications 12 à 16, caractérisé en ce que l'organisme-hôte utilisé pour la production du polypeptide hybride est une bactérie Gram⁻, notamment E. coli.

18. Procédé selon l'une quelconque des revendiCations 12 à 16, caractérisé en ce que l'organisme hôte utilisé pour la production du polypeptide hybride est une bactérie Gram⁺, notamment B. subtilis.

19. Procédé selon l'une quelconque des revendications 12 à 16, caractérisé en ce que l'organisme hôte utilisé pour la production du polypeptide hybride est une cellule eucaryote.

20. Procédé selon l'une quelconque des revendications 17 à 19, caractérisé en ce que le polypeptide déterminé est un épitope, et en ce que la séquence nucléique codant pour cet épitope a été insérée au niveau du site permissif de la séquence codant pour la protéine affine pour le maltose.

21. Composition immunogène dirigée contre des antigènes, notamment des antigènes pathogènes neutralisables par des anticorps dirigés contre un épitope déterminé, caractérisée par l'association du polypeptide hybride obtenu par le procédé selon la revendication 20, et contenant l'épitope déterminé

14

avec un véhicule approprié à la production de compositions de vaccins.

22. Culture de cellules portant au niveau de leur surface externe un épitope caractéristique reconnaissable par le système immunitaire de l'organisme dans lequel les susdites cellules sont susceptibles d'être introduites, caractérisée en ce que lesdites cellules sont tranformées à l'aide d'un acide nucléique recombinant à phase de lecture ouverte comprenant une séquence nucléique codant pour ledit épitope insérée au niveau d'un site permissif d'une séquence nucléique codant pour une protéine affine pour un ose.

23. Culture de cellules selon la revendication 22, caractérisé en ce que la protéine affine pour un ose est la protéine MalE, ou la protéine MalX, et en ce que lesdites cellules sont les souches de bactéries Gram⁻, ou Gram⁺, ou des cellules eucaryotes.

24. Composition immunogène dirigée contre des antigènes, notamment des antigènes pathogènes neutralisables par des anticorps dirigés contre un épitope déterminé, caractérisé par l'association de cellules conformes à la revendication 22 ou 23 avec un véhicule pharmaceutiquement approprié à la production de compositions de vaccin.

25. Acide nucléique recombinant à phase de lecture ouverte comprenant une séquence nucléique codant pour la chaîne peptidique déterminée et une ou plusieurs séquences distinctes codant pour une ou plusieurs parties du polypeptide vecteur, caractérisé en ce que lesdites "une ou plusieurs séquences codant pour une ou plusieurs parties du polypeptide vecteur " sont telles que les propriétés de transport hors du cytoplasme et d'affinité pour l'ose déterminé du polypeptide vecteur intact, sont conservées dans le polypeptide hybride exprimé par ledit acide nucléique recombinant, et en ce que lesdites séquences nucléiques sont sous le contrôle d'éléments de régulation, notamment de la promotion et de la terminaison de la transcription, reconnus par les bactéries Gram⁺.

26. Acide nucléique recombinant à phase de lecture ouverte comprenant une séquence nucléique codant pour la chaîne peptidique déterminée et une ou plusieurs séquences distinctes codant pour une plusieurs parties du polypeptide vecteur, caractérisé en ce que lesdites "une ou plusieurs séquences codant pour une ou plusieurs parties du polypeptide vecteur " sont telles que les propriétés de transport hors du cytoplasme et d'affinité pour l'ose déterminé du polypeptide vecteur intact, sont conservées dans le susdit polypeptide hybride, et en ce que lesdites séquences nucléiques sont sous le contrôle d'éléments de régulation, notamment de la promotion et de la terminaison de la transcription, reconnus par les cellules eucaryotes.

27. Acide nucléique recombinant selon les revendications 25 ou 26, caractérisé en ce que lesdites "une ou plusieurs séquences distinctes codant pour une plusieurs parties du polypeptides vecteur" sont les séquences nucléiques codant pour tout ou partie des protéines MalE ou MalX.

28. Acide nucléique recombinant à phase de lecture ouverte comprenant une séquence nucléique codant pour la chaîne peptidique déterminée et une ou plusieurs séquences peptidiques distinctes codant pour une ou plusieurs parties de la protéine MalX, caractérisé en ce que lesdites "une ou plusieurs séquences codant pour une ou plusieurs parties de MalX" sont telles que les propriétés de transport hors du cytoplasme et d'affinité pour le maltose de la protéine MalX sont conservées dans le polypeptide hybride exprimé par ledit acide nucléique recombinant, et en ce que lesdites séquences nucléiques sont sous le contrôle d'éléments de régulation, notamment de la promotion et de la terminaison de la transcription, reconnus par les bactéries Gram⁻.

29. Acide nucléique recombinant selon la revendication 27 ou 28, caractérisé en ce que la séquence nucléique codant pour MalE et MalX est placée toute entière en amont de la séquence d'ADN codant pour la chaîne peptidique déterminée.

30. Acide nucléique recombinant selon la revendication 27 ou 28, caractérisé en ce que la séquence nucléique codant pour MalE et MalX est placée toute entière en aval de la séquence d'ADN codant pour la chaîne peptidique déterminée.

31. Acide nucléique recombinant selon la revendication 27 ou 28, caractérisé en ce que la séquence nucléique codant pour la chaîne peptidique déterminée est insérée au niveau d'un site permissif de la séquence nucléique codant pour MalE et MalX.

32. Acide nucléique recombinant selon l'une quelconque des revendications 25 à 31, caractérisé en qu'il s'agit d'un vecteur, notamment du type plasmidique.

0299810

FIG. 1

FIG. 2

0299810

Figure 3

Figure 4

pSVME

0
EcoRI
6208

Pst 206

Pvul 5582

PstI 5456

BgII 5329

amp R

SV PolyA

751

988

EcoRI 1068

ß-gl intr

Nco 1573

Bam 1708
Pst 1746
Pvull 1852
Nru/Accl 1865
Bcll 2185
Nco 2266

malE

SVori   TK   Pr

Ndel 4144
Accl 4093
Pvull 3915

Nco 3678
Stu 3590
BgII 3633

HindIII 3569

EcoRI 3444
Pst 3351

BglII 2866
BglII/Bam 3315

Figure 5

1 2 3 4 5 6

- ig

Figure 6

0299810

Figure 7

a

b

Figure 8

FIGURE 9

```
                 10        20        30        40        50        60
MalX  MSSKFMKSTAVLGTVTLASLLLVACGSKTADKPADSGSSEVKELTVYVDEGYKSYIEEVA
         | | ||||| ||    || ||     ||     ||   |  |  |||||| ||||
MalE  MKIKTGARILALSALTMMFSASALAKIEEGKLVIW---------INGDKGYNG-LAEVG
        10        20        30                   40        50

                 70        80        90       100       110       120
      KAYEKEAGVKVTLKTGDALGGLDKLSLDNQSGNVPDVMMAPYDRVGSLGSDGQLSEVKLS
       | || | ||  ||             ||   |||    ||| ||     | |  ||| |
      KKFEKDTGIKVTVEHPDKL--EEKFPQVAATGDGPDIIFWAHDRFGGYAQSGLLAEITPD
        60        70        80        90       100

                130       140       150       160       170
      DGAKTDDTTKSLVTAA-NGKVYGAPAVIESLVMYYNKDLVKDAPKTFADLENLAKDSKYA
      || |||  | |    |  ||      | |   |  |||| |   || |||| |||| |
      KAFQDKLYPFTWDAVRYNGKLIAYPIAVEALSLIYNKDLLPNPPKTWEEIPALDKELK--
      110       120       130       140       150       160

      180       190       200       210       220       230
      FAGEDGKTTAFLADWTNFYYTYGLLAGNGAYVFG-QNGK-DAKDIGLANDGSIAGINYAK
      ||     |||||  | |   ||  ||||||   |   |||   ||| ||||| |||||||
      ---AKGK-SALMFNLQEPYFTWPLIAADGGYAFKYENGKYDIKDVGVDNAGAKAGLTFLV
         170       180       190       200       210       220

      240       250       260       270       280       290
      SWYEKWPKGMQDTEGAGNLIQTQFQEGKTAAIIDGPWKAQAFKDAKVNYGVATIPTLPNG
      |      |||| ||  | ||| |||   | |  |  ||||  |||||   |||  |  |
      D-LIK-NKHM-NADTDYSIAEAAFNKGETAMTINGPWAWSNIDTSKVNYGVTVLPTF-KG
         230       240       250       260       270

      300       310       320       330       340       350
      KEYAAFGGGKAWVIPQAVKNLEASQKFVD-FLVATEQQKVLYDKTNEIPANTEARSYAEG
      |   ||   |   |    |    |    ||||| |||| |||  | | |  ||||  || |
      QPSKPFVGVLSAGINAASPNKELAKEFLENYLLTDEGLEAV-NKDKPL-GAVALKSYEEE
      280       290       300       310       320       330

      360       370       .380
      -KNDELTTAVIKQFKNTQPLPNISQMSA.......(60-80AA)................
       || |||||| |||   | ||||||
      LAKDPRIAATMENAQKGEIMPNIPQMSAFWYAVRTAVINAASGRQTVDEALKDAQTRITK
      340       350       360       370       380       390
```

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | GENE, vol. 29, 1984, pages 27-31, Elsevier Science Publishers; A. ULLMANN: "One-step purification of hybrid proteins which have beta-galactosidase activity" * En entier * --- | 1-24 | C 12 N 15/00 C 12 P 21/02 A 61 K 39/00 C 12 N 1/20 // A 61 K 39/13 C 12 N 9/22 C 12 N 9/12 |
| Y | METHODS IN ENZYMOLOGY, vol. 90, partie E, 1982, pages 459-463, Academic Press Inc., New York, US; O.K. KELLERMANN et al.: "Maltose-binding protein from Escherichia coli" * Page 460, ligne 6 - page 463, ligne 10 * --- | 1-24 | |
| Y | FEBS LETTERS, vol. 94, no. 2, octobre 1978, pages 213-217, Elsevier/North-Holland Biomedical Press; T. FERENCI et al.: "Affinity chromatographic isolation of the periplasmic maltose binding protein of Escherichia coli" * En entier * --- | 1-24 | |
| Y | ANNALES DE MICROBIOLOGIE, vol. 133a, no. 1, 1982, pags 91-100; P.J. BASSFORD, Jr.: "Genetic studies concerning the mechanism of secretion of the maltose-binding protein of Escherichia coli" * En entier * --- | 1-24,25 -32 | |
| A | EP-A-0 157 235 (BAYER AG) --- -/- | | |

DOMAINES TECHNIQUES RECHERCHES (Int. Cl.4)

C 12 N

Le présent rapport a été établi pour toutes les revendications

| Lieu de la recherche | Date d'achèvement de la recherche | Examinateur |
|---|---|---|
| LA HAYE | 06-07-1988 | PULAZZINI A.F.R. |

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| D,A | NATURE, vol. 285, 8 mai 1980, pages 78-81, Macmillan Journals Ltd; H. BEDOUELLE et al.: "Mutations which alter the function of the signal sequence of the maltose binding protein of Escherichia coli"<br>--- | | |
| A | EP-A-0 089 626 (G.D. SEARLE)<br>--- | | |
| A | WO-A-8 404 756 (CELLTECH LTD)<br>--- | | |
| Y | CHEMICAL ABSTRACTS, vol. 98, 1983, page 165, résumé no. 120490e, Columbus, Ohio, US; D.L. STASSI et al.: "Nucleotide sequence of DNA controlling expession of genes for maltossacharide utilization in Streptococcus pneumoniae", & GENE 1982, 20(3), 359-66 * Résumé *<br>--- | 16,27-31 | |
| Y | JOURNAL OF BACTERIOLOGY, vol. 139, no. 1, 1979, pages 19-31, Washington, US; P.J. BASSFORD, Jr. et al.: "Use of gene fusion to study secretion of maltose-binding protein into Escherichia coli periplasm" * En entier *<br>--- | 25-32 | DOMAINES TECHNIQUES RECHERCHES (Int. Cl.4) |
| P,D<br>Y | FR-A-2 595 374 (INSTITUT PASTEUR) * Page 4, ligne 12 - page 9, ligne 28; page 17, lignes 25-33; revendications 9-13 *<br>---         -/- | 20-24 | |

Le présent rapport a été établi pour toutes les revendications

| Lieu de la recherche | Date d'achèvement de la recherche | Examinateur |
|---|---|---|
| LA HAYE | 06-07-1988 | PULAZZINI A.F.R. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Office européen
des brevets

# RAPPORT DE RECHERCHE EUROPEENNE

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| P,X | EUROPEAN JOURNAL OF BIOCHEMISTRY, vol. 171, no. 3, 1 février 1988, pages 541-549, FEBS, 1988; H. BEDOUELLE et al.: "Production in Escherichia coli and one-step purification of bifunctional hybrid proteins which bind maltose" <br> * En entier * <br> ----- | 1-32 | |
| | | | **DOMAINES TECHNIQUES RECHERCHES (Int. Cl.4)** |

Le présent rapport a été établi pour toutes les revendications

| Lieu de la recherche | Date d'achèvement de la recherche | Examinateur |
|---|---|---|
| LA HAYE | 06-07-1988 | PULAZZINI A.F.R. |

CATEGORIE DES DOCUMENTS CITES

X : particulièrement pertinent à lui seul
Y : particulièrement pertinent en combinaison avec un
autre document de la même catégorie
A : arrière-plan technologique
O : divulgation non-écrite
P : document intercalaire

T : théorie ou principe à la base de l'invention
E : document de brevet antérieur, mais publié à la
date de dépôt ou après cette date
D : cité dans la demande
L : cité pour d'autres raisons
························································
& : membre de la même famille, document correspondant

EPO FORM 1503 03.82 (P0402)